# EUROPÄISCHE PATENTSCHRIFT

(11) **EP 3 665 477 B1**
(45) Veröffentlichungstag und Bekanntmachung des Hinweises auf die Patenterteilung: **02.06.2021**
(21) Anmeldenummer: 18746622.2
(22) Anmeldetag: 16.07.2018
(51) Int. Cl.: G01N 33/00, G01M 15/10, G01N 21/64

(54) **GASANALYSATOR ZUR MESSUNG VON STICKOXIDEN UND MINDESTENS EINER WEITEREN KOMPONENTE EINES ABGASES**
GAS ANALYZER FOR MEASURING NITROGEN OXIDES AND AT LEAST ONE FURTHER COMPONENT OF AN EXHAUST GAS
ANALYSEUR DE GAZ POUR MESURER LES OXYDES D'AZOTE ET AU MOINS UN AUTRE CONSTITUANT D'UN GAZ D'ÉCHAPPEMENT

(30) Priorität: 10.08.2017 DE 102017213980
(43) Veröffentlichungstag der Anmeldung: 17.06.2020
(73) Patentinhaber: Siemens Aktiengesellschaft, 80333 München (DE)
(72) Erfinder: HEFFELS, Camiel, 76297 Stutensee-Büchig (DE); SCHMIDT, Benjamin, 76187 Karlsruhe (DE)
(86) Internationale Anmeldenummer: PCT/EP2018/069201
(87) Internationale Veröffentlichungsnummer: WO 2019/029949

(56) Entgegenhaltungen:
- JP-A- H07 225 214
- JP-A- 2004 138 467
- HIGASHI RYOICHI ET AL: "A NOand SOgas analyzer using deep-UV and violet light-emitting diodes for continuous emissions monitoring systems", VISUAL COMMUNICATIONS AND IMAGE PROCESSING, Bd. 9003, 27. Februar 2014 (2014-02-27), Seiten 90031F-1-90031F-6, XP060035256, DOI: 10.1117/12.2036159 ISBN: 978-1-62841-730-2 in der Anmeldung erwähnt

## Beschreibung

Die Erfindung betrifft einen Gasanalysator zur Messung von Stickoxiden und mindestens einer weiteren Komponente eines Abgases
- mit einer Oxidationseinrichtung, die einen Ozongenerator, eine in einem Abgasweg liegende Reaktionskammer sowie eine in dem Abgasweg hinter ihr liegenden Heizkammer aufweist und dazu ausgebildet ist, in der Reaktionskammer das Abgas mit erzeugtem Ozon zu behandeln, um Stickstoffmonoxid in Stickstoffdioxid umzusetzen, und in der Heizkammer Stickoxide und überschüssiges Ozon in Stickstoffdioxid und Sauerstoff thermisch zu zersetzen, und
- mit einem in dem Abgasweg hinter der Oxidationseinrichtung liegenden Photometer, das dazu ausgebildet ist, anhand der Absorption von Licht im nahen Ultraviolett-Bereich zwischen 350 nm und 500 nm in dem behandelten Abgas die Stickstoffdioxid-Konzentration zu ermitteln und als Stickoxid-Konzentration des unbehandelten Abgases auszugeben.

Aus Ryoichi Higashi et al.: "A NOx and SO2 gas analyzer using deep-UV and violet light-emitting diodes for continuous emissions monitoring systems", Proc. SPIE 9003, Light-Emitting Diodes: Materials, Devices, and Applications for Solid State Lighting XVIII, 90031F (February 27, 2014), ist ein Gasanalysator mit einem Photometer zur Messung von Stickoxiden und Schwefeldioxid in einem Abgas bekannt. Vor der Analyse wird das Abgas in zwei Stufen behandelt, wobei in einer ersten Stufe in dem Abgas enthaltenes Stickstoffmonoxid mit Hilfe von Ozon in mit dem Photometer messbares Stickstoffdioxid umgesetzt wird. Das Ozon wird mittels elektrischer Entladung aus Luftsauerstoff erzeugt und dem Abgas zugeführt. In einer zweiten Behandlungsstufe wird das Abgas auf etwa 300 °C erhitzt, um überschüssiges Ozon und durch Reaktion von Stickstoffdioxid und Ozon entstandenes und mit dem Photometer nicht messbares Distickstoffpentoxid (N2O5) thermisch in Stickstoffdioxid zu zersetzen. Die von dem Gasanalysator ermittelte Konzentration von Stickstoffdioxid ist somit ein Maß für die Konzentration von Stickoxiden in dem Abgas.

In dem Photometer des bekannten Gasanalysators sind eine erste Leuchtdiode mit einer Emissionswellenlänge von 280 nm und eine zweite Leuchtdiode mit einer Emissionswellenläge von 400 nm dicht nebeneinander in einem LED-Array angeordnet. Ihr Licht wird mittels einer Kollimatorlinse zu einem parallelen Lichtbündel geformt, das eine von dem behandelten Abgas durchströmte Messkammer durchstrahlt und anschließend auf einen Detektor fokussiert wird. Mittels eines Strahlteilers zwischen Kollimatorlinse und Messkammer wird ein Teil des Lichts auf einen Monitordetektor gelenkt. Die Leuchtdioden werden im Wechsel ein- und ausgeschaltet, um das in dem Abgas enthaltene Schwefeldioxid bei der Absorptionswellenlänge 280 nm und Stickstoffdioxid bei der Absorptionswellenlänge 400 nm zu detektieren. Das Detektorsignal wird mit dem Signal des Monitordetektors normalisiert, bevor es zur Ermittlung der Schwefeldioxid- und Stickstoffdioxid- bzw. Stickoxid-Konzentrationen in dem Abgas ausgewertet wird. Die Temperatur der Leuchtdioden wird mittels eines Peltierelements auf einen konstanten Wert geregelt.

Aus der US 5 806 305 A ist eine Einrichtung zur katalytischen Abgasnachbehandlung bei Brennkraftmaschinen (Otto- oder Dieselmotoren) bekannt, bei der dem Abgas vor der Behandlung in dem Katalysator Ozon zugeführt wird. Das Ozon wird in einem Ozongenerator aus Frischluft mittels UV-Licht, beispielsweise einer Quecksilberdampflampe, bei einer Wellenlänge von 185 nm erzeugt.

Aus der EP 1 020 620 B1 ist es bekannt, zu demselben Zweck das Ozon aus Frischluft oder dem Abgas mittels UV-Licht, Mikrowelle oder Funkenentladung zu erzeugen.

Aus der EP 2 157 421 A1 ist es bekannt, zur Untersuchung des Schwefelgehalts von Kraftstoffen eine Probe des Kraftstoffs zu verbrennen und die Konzentration von Schwefeldioxid in dem Abgas durch ein Ultraviolett-Fluoreszenz-Messverfahren zu bestimmen. Um die Beeinträchtigung der Messung durch Stickstoffmonoxid zu vermeiden, wird das Abgas zuvor in einem Behälter dem Licht (185 nm) einer Niederdruck-Quecksilberentladungslampe ausgesetzt, um aus dem Restsauerstoffgehalt Ozon zu erzeugen und damit Stickstoffmonoxid in Stickstoffdioxid umzusetzen.

Der Erfindung liegt die Aufgabe zugrunde, zusammen mit der Messung von Stickoxiden in Abgasen auch eine Bestimmung der Sauerstoff-Konzentration zu ermöglichen.

Gemäß der Erfindung wird die Aufgabe dadurch gelöst, dass bei dem Gasanalysator der eingangs angegebenen Art
- der Ozongenerator eine in der Reaktionskammer angeordnete Ultraviolett-Lichtquelle umfasst und dazu ausgebildet ist, das Ozon aus dem Restsauerstoffgehalt des Abgases zu erzeugen, und
- in dem Abgasweg zwischen der Reaktionskammer und der Heizkammer ein weiteres Photometer angeordnet ist, das dazu ausgebildet ist, anhand der Absorption von Licht im mittleren Ultraviolett-Bereich zwischen 220 nm und 300 nm in dem teilbehandelten Abgas dessen Ozon-Konzentration zu ermitteln und mithilfe eines Proportionalitätsfaktors die Sauerstoff-Konzentration des unbehandelten Abgases zu berechnen und auszugeben.

Vorteilhafte Weiterbildungen des erfindungsgemäßen Gasanalysators sind in den Unteransprüchen angegeben.

Die in der Publikation von Ryoichi Higashi et al. erwähnte Ozonerzeugung aus Luftsauerstoff mittels elektrischer Entladung (Koronaentladung) hat den Nachteil, dass sich Stickoxidverbindungen bilden, die für die Stickoxid-Messung des Abgases unerwünscht sind. Die Oxidationseinrichtung des erfindungsgemäßen Gasanalysators weist daher vorzugsweise einen Ozongenerator mit einer hochenergetische Strahlung mit einer Wellenlänge kleiner als 240 nm erzeugenden Ultraviolett-Lichtquelle, beispielsweise eine elektrodenlose Excimerlampe oder eine Niederdruck-Quecksilberentladungslampe, in der von dem Abgas durchströmten Reaktionskammer auf. Bei magerer Verbrennung mit Luftüberschuss kann so das Ozon vollständig aus dem Restsauerstoffgehalt des Abgases erzeugt werden. Die Reaktionskammer kann z. B. in Form eines innenverspiegelten Rohres (z. B. Aluminium-Rohr) ausgebildet sein, durch das sich die beispielsweise zylinderförmige Ultraviolett-Lichtquelle erstreckt. Zur Erhöhung der Leistung bei der Umsetzung von Stickstoffmonoxid in Stickstoffdioxid können mehrere solcher Rohre parallel geschaltet sein. Wenn die Leistung der Ultraviolett-Lichtquelle mit der Zeit nachlässt, kann dem Abgas zusätzlich Luftsauerstoff zugeführt werden.

In der Reaktionskammer findet die Umsetzung von Sauerstoff in Ozon proportional zu der in dem Abgas vorhandenen Sauerstoff-Konzentration und der Ultraviolett-Lichtleistung statt. Bei konstantem Durchfluss des Abgases (und damit konstanter Verweilzeit in der Reaktionskammer) ist daher die Ozon-Konzentration des teilbehandelten Abgases beim Verlassen der Reaktionskammer ein Maß für die Sauerstoff-Konzentration des unbehandelten Abgases. Das weitere Photometer misst die Ozon-Konzentration anhand der Lichtabsorption im mittleren Ultraviolett-Bereich zwischen 220 nm und 300 nm und gibt diese als Sauerstoff-Konzentration des unbehandelten Abgases aus. Der Proportionalitätsfaktor des praktisch linearen Zusammenhangs zwischen der Sauerstoff-Konzentration und der Ozon-Konzentration kann durch Kalibration sehr einfach ermittelt werden.

Die Querempfindlichkeit der Schwefeldioxid-Konzentration auf den von dem weiteren Photometer erzeugten Messwert für die Ozon- bzw. Sauerstoff-Konzentration wird rechnerisch kompensiert. Die Querempfindlichkeit der Stickstoffdioxid-Konzentration auf den von dem Photometer erzeugten Messwert für die Schwefeldioxid-Konzentration wird ebenfalls rechnerisch kompensiert.

Wenn mit Hilfe eines Bypasses das Abgas an der Oxidationseinrichtung vorbei zu dem Photometer geleitet wird, erhält man die Stickstoffdioxid-Konzentration des unbehandelten Abgases. Aus der Differenz der in dem behandelten Abgas und dem unbehandelten Abgas gemessenen Stickstoffdioxid-Konzentrationen ergibt sich die Stickstoffmonoxid-Konzentration des unbehandelten Abgases, die auch ein Maß für den Ozonverbrauch in der Oxidationseinrichtung bei der Umsetzung von Stickstoffmonoxid in Stickstoffdioxids ist. Das Ergebnis für die Sauerstoff-Konzentration des unbehandelten Abgases wird daher um den Betrag der ermittelten Stickstoffmonoxid-Konzentration korrigiert.

Da die Ultraviolett-Leistung des Leuchtmittels im Ozongenerator mit der Zeit nachlässt, kann es erforderlich sein, dem Gasanalysator regelmäßig Umgebungsluft mit 21 Vol-% Sauerstoff zuzuführen. Damit werden der Endwert der Ozon-Messung und ebenfalls der Nullpunkt der Schwefeldioxid- und Stickstoffdioxid Messung der beiden Photometer wieder neu eingestellt.

Wenn, wie im Falle des An- und Abfahrbetriebs der Verbrennungsmaschine, eine hohe Stickstoffdioxid-Konzentration im Abgas vorliegt, kann statt des Dreiwegeventils auch ein parallel zu den beiden Photometern geschaltetes drittes Photometer für die Messung der Stickstoffdioxid-Konzentration im Abgas vorgesehen werden.

Im Weiteren wird die Erfindung anhand von Beispielen erläutert, wozu auf die Figuren der Zeichnung Bezug genommen wird; im Einzelnen zeigen:
- Figur 1: ein Blockschaltbild des erfindungsgemäßen Gasanalysators zur Messung von Stickoxiden, Schwefeldioxid und Sauerstoff,
- Figur 2: ein Ausführungsbeispiel des Ozongenerators,
- Figur 3: beispielhaft die Absorptionsspektren von Stickstoffdioxid, Schwefeldioxid und Ozon sowie die Zentralwellenlängen der Emissionsspektren der Leuchtdioden zur Messung dieser Komponenten, und
- Figur 4: ein detaillierteres Ausführungsbeispiel des Gasanalysators.

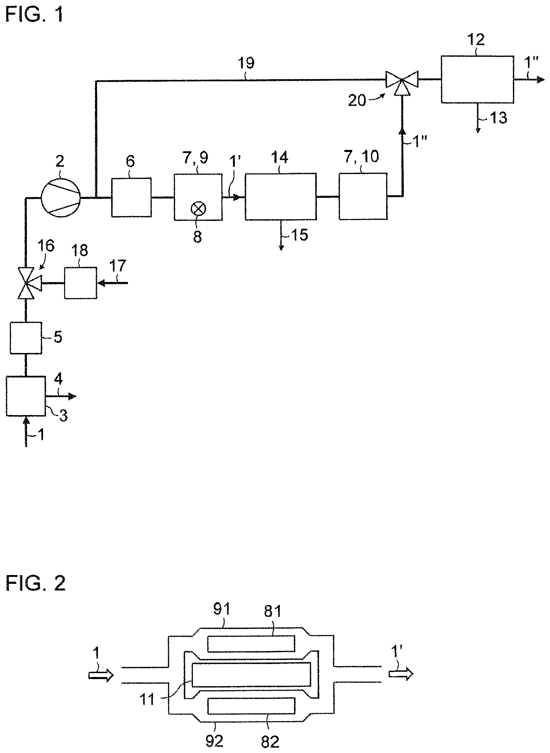

Figur 1 zeigt in vereinfachter schematischer Darstellung ein Blockschaltbild eines Gasanalysators zur Messung von Stickoxiden, Schwefeldioxid und Sauerstoff in einem Abgas 1. Das Abgas 1 wird mittels einer Messgaspumpe 2 von einer Probenahmestelle über einen Gaskühler 3 mit Kondensatauslass 4 und ein Feinpartikelfilter 5 angesaugt. Der Gaskühler 3 dient dazu, ein Auskondensieren von Wasser in dem Gasanalysator zu verhindern. Der Durchfluss des Abgases 1 wird mit Hilfe eines Durchflussreglers 6 in Form eines Massendurchflussreglers oder Druckreglers mit anschließender Drossel konstant eingestellt.

Um die Messung der Stickoxid-Konzentration zu ermöglichen, wird das Abgas 1 in einer zweistufigen Oxidationseinrichtung 7 behandelt, die in einer ersten Stufe einen Ozongenerator in Form einer Ultraviolett-Lichtquelle 8 in einer von dem Abgas 1 durchströmten geschlossenen Reaktionskammer 9 und in einer zweiten Stufe eine Heizkammer 10 aufweist. Die Ultraviolett-Lichtquelle 8, bei der es sich beispielsweise um eine elektrodenlose Excimerlampe oder eine Niederdruck-Quecksilberentladungslampe handelt, erzeugt mit ihrer Strahlung von beispielsweise 185 nm aus dem Restsauerstoff des Abgases 1 Ozon, welches in der Reaktionskammer 9 in dem Abgas 1 enthaltenes Stickstoffmonoxid in Stickstoffdioxid umsetzt. Die erzeugte Ozonmenge ist proportional zu dem Sauerstoffgehalt des Abgases 1 und der UV-Leistung der Lichtquelle 8. Der konstante Durchfluss des Abgases 1 ist wichtig, weil die Ozonproduktion auch von der Verweilzeit des Sauerstoffs im Ozongenerator abhängt und bei zu großem Massenstrom abnimmt. Das vorhandene bzw. umgesetzte Stickstoffdioxid kann jedoch wieder mit dem erzeugten Ozon reagieren, wobei weitere Stickoxide, vor allem Distickstoffpentoxid, entstehen. In der Heizkammer (Gasheizer) 10 werden diese unerwünschten Stickoxide sowie überschüssiges Ozon bei etwa 300 °C thermisch zu Stickstoffdioxid und Sauerstoff zersetzt.

Figur 2 zeigt sehr vereinfacht ein Ausführungsbeispiel des Ozongenerators mit hier zwei Ultraviolett-Lichtquellen 81, 82 in Form von zylinderförmigen Niederdruck-Quecksilberentladungslampen die in zwei parallel geschalteten und von dem Abgas 1 durchströmten innenverspiegelten Aluminium-Rohren 91, 92 angeordnet sind. Die Rohre 71, 72 bilden die Reaktionskammer 9. Die Anregung des Quecksilberplasmas kann über Elektroden oder, zur Erhöhung der Lebensdauer, elektrodenlos mittels eines Mikrowellengenerators 11 erfolgen.

Zurück zu Figur 1 wird das behandelte Abgas 1" nach Verlassen der Heizkammer 10 einem Photometer 12 zugeführt, das die Konzentrationen von Stickstoffdioxid und Schwefeldioxid anhand der Absorption von Licht jeweils im Bereich der Wellenlängen von 405 nm und 285 nm misst. Da die Stickstoffdioxid-Konzentration des behandelten Abgases 1" die Summe aus der Stickstoffdioxid-Konzentration und der Konzentration des zu Stickstoffdioxid umgesetzten Stickstoffmonoxids des unbehandelten Abgases 1 ist, gibt das Photometer 12 als Analysenergebnis 13 neben der Schwefeldioxid-Konzentration die Stickoxid-Konzentration des zu analysierenden Abgases 1 aus.

In dem Abgasweg zwischen der Reaktionskammer 9 der Oxidationseinrichtung 7 und der Heizkammer 10 ist ein weiteres Photometer 14 angeordnet, das die Konzentrationen von Ozon in dem aus der Reaktionskammer 9 kommenden teilbehandelten Abgas 1' anhand der Absorption von Licht im Bereich der Wellenlänge von 285 nm misst. Da, wie bereits erwähnt, die von der Ultraviolett-Lichtquelle 8 in der Reaktionskammer 9 erzeugte Ozonmenge proportional zu dem Sauerstoffgehalt des Abgases 1 ist, gibt das weitere Photometer 14 als Analysenergebnis 15 die Sauerstoff-Konzentration des zu analysierenden Abgases 1 aus.

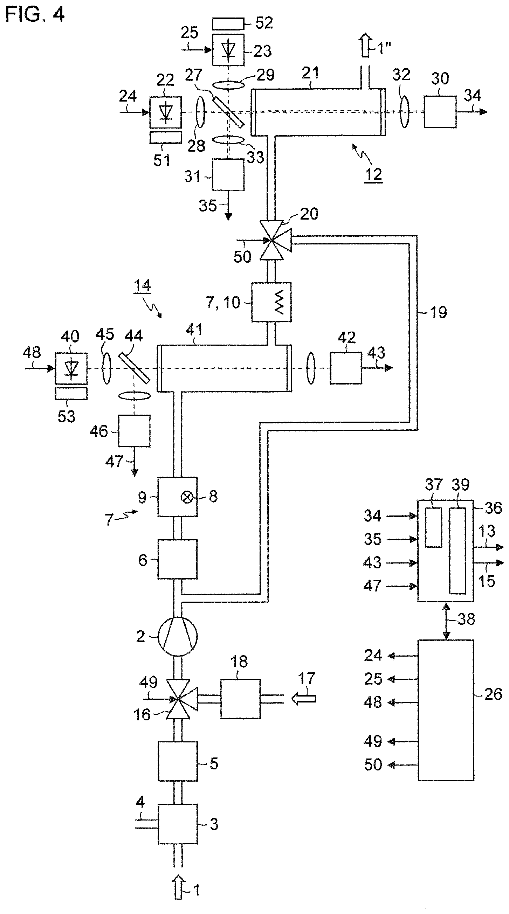

Wie weiter unten anhand der Figur 4 erläutert wird, handelt es sich bei den beiden Photometern 12, 14 um LED-Photometer mit Leuchtdioden.

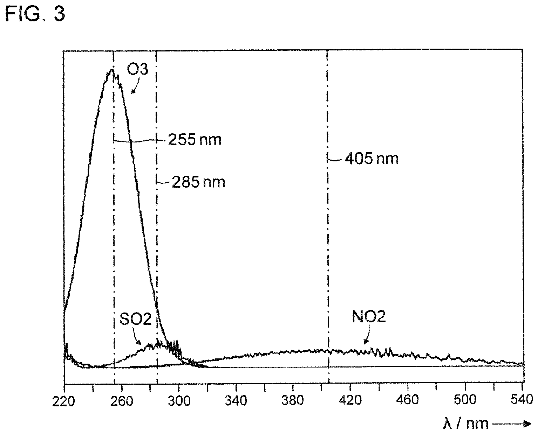

Figur 3 zeigt beispielhaft die Absorptionsspektren von Stickstoffdioxid NO2, Ozon O3 und Schwefeldioxid SO2 sowie die hier durch die Zentralwellenlängen 405 nm, 255 nm und 285 nm repräsentierten Emissionsspektren der Leuchtdioden zur Messung der genannten Komponenten.

Zurück zu Figur 1 ist in dem Abgasweg vor der Oxidationseinrichtung 7 ist ein steuerbares Dreiwege-Mischventil 16 angeordnet, um bei Bedarf dem Abgas 1 Luftsauerstoff 17 beimischen zu können, der über ein Feinpartikelfilter 18 von der Messgaspumpe 2 angesaugt wird. Die Beimischung kann manuell oder automatisch durch das weitere Photometer 14 gesteuert erfolgen wenn der Restsauerstoffgehalt des Abgases 1 oder die Leistung des Ozongenerators 8 für die zur vollständigen Umsetzung von Stickstoffmonoxid in Stickstoffdioxid erforderliche Ozonerzeugung nicht ausreicht.

Zwischen der Messgaspumpe 2 und dem Photometer 12 ist ein Bypass 19 vorgesehen, der über ein steuerbares Dreiwegeventil 20 zur Umgehung der Oxidationseinrichtung 7 einschaltbar ist. Bei eingeschaltem Bypass 19 gelangt das unbehandelte Abgas 1 in das Photometer 12, so dass von diesem die Stickstoffdioxid-Konzentration des Abgases 1 anstelle der Stickoxid-Konzentration gemessen wird. Da bei stationärem Verbrennungsprozess die Stickstoffdioxid-Konzentration im Abgas 1 generell gering ist, ist im Normalfall der Bypass 19 abgeschaltet, so dass die Stickoxid-Konzentration gemessen wird. Jedoch kann bei geringem Restsauerstoffgehalt des Abgases 1 die Stickstoffmonoxid-Konzentration im Abgas 1 benötigt werden, um den Ozonverlust in der Reaktionskammer 9 durch die Oxidation des Stickstoffmonoxids bei der Ozonerzeugung in die Berechnung der Sauerstoff-Konzentration zu berücksichtigen. Die Stickstoffmonoxid-Konzentration ergibt sich dann aus der Differenz der in dem behandelten Abgas 1" und dem unbehandelten Abgas 1 gemessenen Stickstoffdioxid-Konzentrationen. Dieser Differenz entspricht der Anteil des in dem behandelten Abgas 1" enthaltenen Stickstoffdioxids, der aus der Umsetzung von Stickstoffmonoxid mit Ozon entstanden ist, und daher von dem weiteren Photometer 14 nicht gemessen wird, und ist daher ein Maß für den Ozonverlust, um den das von dem weiteren Photometer 14 ausgegebene Ergebnis 15 für die Sauerstoff-Konzentration daher um den Betrag der ermittelten Stickstoffmonoxid-Konzentration korrigiert wird.

Wenn, wie im Falle des An- und Abfahrbetriebs der Verbrennungsmaschine, eine hohe Stickstoffdioxid-Konzentration im Abgas 1 vorliegt, kann statt des Dreiwegeventil 20 auch ein parallel zu den beiden Photometern 12, 14 geschaltetes drittes Photometer für die Messung der Stickstoffdioxid-Konzentration im Abgas 1 vorgesehen werden.

Figur 4 zeigt ein detaillierteres Ausführungsbeispiel des erfindungsgemäßen Gasanalysators. Wie auch schon in Figur 1 gezeigt, wird das Abgas 1 wird mittels der Messgaspumpe 2 von über den Gaskühler 3 mit Kondensatauslass 4 und das Feinpartikelfilter 5 angesaugt, wobei der Durchfluss des mit Hilfe des Durchflussreglers 6 konstant eingestellt wird. Das Abgas 1 wird in der Oxidationseinrichtung 7 behandelt, wobei es zuerst die Reaktionskammer 9 mit der Ultraviolett-Lichtquelle 8 und danach die Heizkammer 10 durchläuft. Das nachgeordnete Photometer 12 misst die Stickstoffdioxid- und Schwefeldioxid-Konzentrationen des behandelten Abgases 1". Das weitere Photometer 14 misst die Konzentrationen von Ozon des teilbehandelten Abgases 1' im Abgasweg zwischen der Reaktionskammer 9 und der Heizkammer 10. Das in dem Abgasweg vor der Oxidationseinrichtung 7 liegende Dreiwege-Mischventil 16 ermöglicht die Beimischung von Luftsauerstoff 17, der von der Messgaspumpe 2 über das Feinpartikelfilter 18 angesaugt wird. Der über das Dreiwegeventil 20 einschaltbare Bypass 19 ermöglicht es, das unbehandelte Abgas 1 unter Umgehung der Oxidationseinrichtung 7 direkt in das Photometer 12 zu leiten.

Das Photometer 12 weist eine Messkammer 21 auf, durch die das behandelte Abgas 1" geleitet wird und die von dem Licht einer ersten Leuchtdiode 22 mit der Zentralwellenlänge 405 nm und einer zweiten Leuchtdiode 23 mit der Zentralwellenlänge 285 nm durchstrahlt wird. Die beiden Leuchtdioden 22, 23 werden mit Steuersignalen 24, 25 einer Steuereinrichtung 26 unterschiedlich angesteuert, beispielsweise abwechselnd ein- und ausgeschaltet oder unterschiedlich moduliert, z. B. mit unterschiedlichen Modulationsfrequenzen, Taktraten oder Pulse-Codes. Die beiden Leuchtdioden 22, 23 können z. B. dicht nebeneinander in einem LED-Array angeordnet sein. Bei dem gezeigten Beispiel sind die Leuchtdioden 22, 23 gegenüber verschiedenen Seiten eines Strahlteilers 27 angeordnet, der das von Linsen 28, 29 kollimierte Licht der Leuchtdioden 22, 23 in einen Teilstrahl durch die Messkammer 21 hindurch auf einen Detektor 30 und einen weiteren Teilstrahl auf einen Referenzdetektor 31 aufteilt. Die beiden Teilstrahlen können mit Hilfe von Linsen 32, 33 auf die Detektoren 22, 23 fokussiert werden, bei denen es sich hier um Photodioden handelt.

Der Detektor 30 erzeugt ein Detektorsignal 34 mit einem aus dem Licht der ersten Leuchtdiode 22 resultierenden ersten Signalanteil und einem aus dem Licht der zweiten Leuchtdiode 23 resultierenden zweiten Signalanteil. Der Referenzdetektor 31 erzeugt ein Referenzsignal 35 mit aus dem Licht beider Leuchtdioden 22, 23 resultierenden Referenz-Signalanteilen. Das Detektorsignal 34 und das Referenzsignal 35 werden einer Auswerteeinrichtung 36 zugeführt, die einen Demultiplexer oder Demodulator 37 enthält, um die verschiedenen Signalanteile des Detektorsignals 34 und des Referenzsignals 35 für die weitere Verarbeitung und Auswertung zu trennen. Die Synchronisierung der unterschiedlichen Ansteuerung der Leuchtdioden 22, 23 und der Detektion der unterschiedlichen Signalanteile erfolgt über eine Kommunikationsleitung 38 zwischen der Steuereinrichtung 26 und der Auswerteeinrichtung 36. Nach Aufbereitung, z. B. Filterung und Digitalisierung, der Signale 34, 35 werden in einer Recheneinrichtung 39 aus dem ersten Signalanteil des Detektorsignals 34 die Stickstoffdioxid-Konzentration des behandelten Abgases 1" und aus dem zweiten Signalanteil die Schwefeldioxid-Konzentration ermittelt. Dabei werden die Signalanteile des Detektorsignals 34 mit den zugehörigen Signalanteilen des Referenzsignals 35 referenziert, so dass das ausgegebene Analysenergebnis 13 von der Helligkeit der Leuchtdioden 22, 23 und damit z. B. von ihrem Alterungszustand unabhängig ist. In dem Analysenergebnis 13 wird die Stickstoffdioxid-Konzentration des behandelten Abgases 1" als die Stickoxid-Konzentration des unbehandelten Abgases 1 ausgegeben.

In dem weiteren Photometer 14 wird das Licht mit der Zentralwellenlänge 255 nm einer dritten Leuchtdiode 40 durch eine weitere Messkammer 41 hindurch auf einen weiteren Detektor 42 geleitet, der ein weiteres Detektorsignal 43 erzeugt. Auch hier teilt ein Strahlteiler 44 das von einer Linse 45 kollimierte Licht in einen Teilstrahl durch die weitere Messkammer 41 und einen weiteren Teilstrahl auf einen weiteren Referenzdetektor 46 auf. Die Auswerteeinrichtung 36 ermittelt aus dem weiteren Detektorsignal 43 und dem Referenzsignal 47 des weiteren Referenzdetektors 46 die Ozon-Konzentration des teilbehandelten Abgases 1', um dies in dem Analysenergebnis 15 als die Sauerstoff-Konzentration unbehandelten des Abgases 1 auszugeben. Die dritte Leuchtdiode 40 kann ebenfalls mit einem Steuersignal 48 der Steuereinrichtung 26 angesteuert werden, um z. B. das erzeugte Licht zu modulieren und so die Messung gegenüber Störlichteinflüssen unempfindlich zu machen. Die Steuereinrichtung 26 erzeigt auch Steuersignale 49, 50 für die Ventile 16, 20.

Trotz der erwähnten Referenzierung der Messungen ist es vorteilhaft, die gesamte photometrische Messanordnung des Gasanalysators zu thermostatieren. Dazu gehört auch eine Thermostatisierung der Leuchtdioden 22, 23, 40 mit Hilfe von Peltierelementen 51, 52, 53, um Messbereiche im unteren ppm-Bereich realisieren zu können.

Anstelle der gezeigten gemeinsamen Auswerteeinrichtung 36 kann jeder der beiden Photometer 12, 14 eine eigene Auswerteeinrichtung aufweisen, welche miteinander kommunizieren.

Der gezeigte Gasanalysator kann ohne Weiteres für die Messung weiterer Bestandteile des Abgases 1, wie z. B. Kohlendioxid, Kohlenmonoxid, Schwefel-, Chlor- und Iod-Verbindungen, erweitert werden. Anstelle der dazu erforderlichen weiteren geeigneten Lichtquellen (z. B. Leuchtdioden) und ggf. weiterer Strahlteiler in dem Photometer 12 können einzelne bereits vorhandene Leuchtdioden mit einem Leuchtstoff (Phosphor) versehen werden, der das von der betreffenden Leuchtdiode erzeugte Licht teilweise in ein Licht mit größerer Wellenlänge umwandelt. Dieses Prinzip ist beispielsweise aus der US 2010/049017 A1 bekannt.

## Patentansprüche

1. Gasanalysator zur Messung von Stickoxiden und mindestens einer weiteren Komponente eines Abgases (1)
- mit einer Oxidationseinrichtung (7), die einen Ozongenerator (8), eine in einem Abgasweg liegende Reaktionskammer (9) und eine in dem Abgasweg hinter ihr liegenden Heizkammer (10) aufweist und dazu ausgebildet ist, in der Reaktionskammer (9) das Abgas (1) mit erzeugtem Ozon zu behandeln, um Stickstoffmonoxid in Stickstoffdioxid umzusetzen, und in der Heizkammer (10) Stickoxide und überschüssiges Ozon in Stickstoffdioxid und Sauerstoff thermisch zu zersetzen, und
- mit einem in dem Abgasweg hinter der Oxidationseinrichtung (7) liegenden Photometer (12), das dazu ausgebildet ist, anhand der Absorption von Licht im nahen Ultraviolett-Bereich zwischen 350 nm und 500 nm in dem behandelten Abgas (1") die Stickstoffdioxid-Konzentration zu ermitteln und als Stickoxid-Konzentration des unbehandelten Abgases (1) auszugeben,
**dadurch gekennzeichnet,**
- **dass** der Ozongenerator (8) eine in der Reaktionskammer (9) angeordnete Ultraviolett-Lichtquelle (8) umfasst und dazu ausgebildet ist, das Ozon aus dem Restsauerstoffgehalt des Abgases (1) zu erzeugen, und
- **dass** in dem Abgasweg zwischen der Reaktionskammer (9) und der Heizkammer (10) ein weiteres Photometer (14) angeordnet ist, das dazu ausgebildet ist, anhand der Absorption von Licht im mittleren Ultraviolett-Bereich zwischen 220 nm und 300 nm in dem teilbehandelten Abgas (1') dessen Ozon-Konzentration zu ermitteln und mithilfe eines Proportionalitätsfaktors die Sauerstoff-Konzentration des unbehandelten Abgases (1) zu berechnen und auszugeben.

2. Gasanalysator nach Anspruch 1, **dadurch gekennzeichnet, dass** das Photometer (12) und das weitere Photometer (14) jeweils eine von dem Abgas (1" ; 1') durchströmte Messkammer (21; 41), eine das Licht erzeugende Leuchtdiode (22; 40), einen Detektor (30; 42), einen Referenzdetektor (31; 46), eine Auswerteeinrichtung (36) und einen Strahlteiler (27; 44) aufweisen, der dazu ausgebildet ist, einen Teil des Lichts der Leuchtdioden (22; 40) durch die Messkammer (21; 41) hindurch auf den Detektor (30; 46) und den anderen Teil des Lichts auf den Referenzdetektor (31; 46) zu führen, und dass die Auswerteeinrichtung (36) das Detektorsignal (34; 43) mit dem Referenzsignal (35; 47) referenziert.

3. Gasanalysator nach Anspruch 1 oder 2, **dadurch gekennzeichnet, dass** das Photometer (12) ferner dazu ausgebildet ist, anhand der Absorption von Licht im mittleren Ultraviolett-Bereich zwischen 250 nm und 300 nm in dem Abgas (1") die Schwefeldioxid-Konzentration des Abgases (1) zu ermitteln und auszugeben.

4. Gasanalysator nach Anspruch 2 und 3, **dadurch gekennzeichnet,**
- **dass** das Photometer (12) eine zweite, das Licht im mittleren Ultraviolett-Bereich zwischen 350 nm und 500 nm erzeugende Leuchtdiode (23) aufweist,
- **dass** der Strahlteiler (27) dazu ausgebildet ist, einen Teil des Lichts der zweiten Leuchtdiode (23) durch die Messkammer (21) hindurch auf den Detektor (30) und den anderen Teil des Lichts auf den Referenzdetektor (31) zu führen,
- **dass** eine Steuereinrichtung (26) vorhanden ist, die die erste Leuchtdiode (22) und die zweite Leuchtdiode (23) unterschiedlich anzusteuern, so dass das Detektorsignal (34) und das Referenzsignal (35) jeweils aus dem Licht der ersten Leuchtdiode (22) und dem Licht der zweiten Leuchtdiode (23) resultierende unterschiedliche Signalanteile enthalten, und
- **dass** die Auswerteeinrichtung (28) aus dem aus dem Licht der ersten Leuchtdiode (22) resultierenden Signalanteil des Detektorsignals (34) die Stickstoffdioxid-Konzentration und aus dem Licht der zweiten Leuchtdiode (22) resultierenden Signalanteil die Schwefeldioxid-Konzentration des Abgases (1") ermittelt.

5. Gasanalysator nach einem der Ansprüche 2 bis 4, **dadurch gekennzeichnet, dass** die Photometer (12, 14) zwei miteinander kommunizierende Auswerteeinrichtungen oder eine gemeinsame Auswerteeinrichtung (36) haben, die dazu ausgebildet ist, die Querempfindlichkeit der ermittelten Schwefeldioxid-Konzentration des Abgases (1") gegenüber Stickstoffdioxid mit der ermittelten Stickstoffdioxid-Konzentration des Abgases (1'') zu korrigieren.

6. Gasanalysator nach Anspruch 5, **dadurch gekennzeichnet, dass** die Auswerteeinrichtung (36) dazu ausgebildet ist, die Querempfindlichkeit der ermittelten Ozon-Konzentration des Abgases (1') gegenüber Schwefeldioxid mit der ermittelten Schwefeldioxid-Konzentration des Abgases (1") zu korrigieren.

7. Gasanalysator nach einem der vorangehenden Ansprüche, **dadurch gekennzeichnet, dass** ein einen steuerbarer Bypass (19) zur Umgehung der Oxidationseinrichtung (7) vorhanden ist, dass das Photometer (12) bei aktiviertem Bypass (19) die Stickstoffdioxid-Konzentration in dem unbehandelten Abgas (1) misst und den Unterschied gegenüber der für das behandelte Abgas (1") ermittelten Stickstoffdioxid-Konzentration als die Stickstoffmonoxid-Konzentration des unbehandelten Abgases (1) ausgibt.

8. Gasanalysator nach Anspruch 7, **dadurch gekennzeichnet, dass** die Auswerteeinrichtung (36) dazu ausgebildet ist, das von dem weiteren Photometer (14) gelieferte Ergebnis (15) der Ozon-Konzentration bzw. der Sauerstoff-Konzentration des unbehandelten Abgases (1) mit der ermittelten Stickstoffmonoxid-Konzentration des unbehandelten Abgases (1) zu korrigieren.

## Claims

1. Gas analyser for measuring nitrogen oxides and at least one further component of an exhaust gas (1)
- with an oxidation device (7) comprising an ozone generator (8), a reaction chamber (9) located in an exhaust gas path and a heating chamber (10) located downstream thereof in the exhaust gas path and which is embodied to treat the exhaust gas (1) with generated ozone in the reaction chamber (9) in order to convert nitrogen monoxide into nitrogen dioxide and to induce thermal decomposition of nitrogen oxides and excess ozone into nitrogen dioxide and oxygen in the heating chamber (10) and
- with a photometer (12) located downstream of the oxidation device (7) in the exhaust gas path, which is embodied to ascertain the nitrogen dioxide concentration based on the absorption of light in the near-ultraviolet range between 350 nm and 500 nm in the treated exhaust gas (1") and output the same as the nitrogen oxide concentration of the untreated exhaust gas (1),
**characterised in**
- **that** the ozone generator (8) includes an ultraviolet light source (8) arranged in the reaction chamber (9) and is embodied to generate the ozone from the residual oxygen content of the exhaust gas (1) and
- **that**, arranged in the exhaust gas path between the reaction chamber (9) and the heating chamber (10), there is a further photometer (14), which is embodied to ascertain the ozone concentration in the partially treated exhaust gas (1') based on the absorption of light in the mid-ultraviolet ultraviolet range between 220 nm and 300 nm and, with the aid of a proportionality factor, to calculate and output the oxygen concentration of the untreated exhaust gas (1).

2. Gas analyser according to claim 1, **characterised in that** the photometer (12) and the further photometer (14) each comprise a measuring chamber (21; 41) through which the exhaust gas (1"; 1') flows, a light-emitting diode (22; 40) generating the light, a detector (30; 42), a reference detector (31; 46), an evaluation device (36) and a beam splitter (27; 44), which is embodied to guide a part of the light from the light-emitting diodes (22; 40) through the measuring chamber (21; 41) to the detector (30; 46) and the other part of the light to the reference detector (31; 46) and that the evaluation device (36) references the detector signal (34; 43) with the reference signal (35; 47).

3. Gas analyser according to claim 1 or 2, **characterised in that** the photometer (12) is further embodied to ascertain the sulfur dioxide concentration of the exhaust gas (1) based on the absorption of light in the mid-ultraviolet range between 250 nm and 300 nm in the exhaust gas (1") and output the same.

4. Gas analyser according to claim 2 and 3, **characterised in**
- **that** the photometer (12) comprises a second light-emitting diode (23) generating the light in the mid-ultraviolet range between 350 nm and 500 nm,
- **that** the beam splitter (27) is embodied to guide a part of the light from the second light-emitting diode (23) through the measuring chamber (21) to the detector (30) and the other part of the light to the reference detector (31),
- **that** a control device (26) is present which actuates the first light-emitting diode (22) and the second light-emitting diode (23) differently so that the detector signal (34) and the reference signal (35) each contain different signal components resulting in each case from the light from the first light-emitting diode (22) and the light from the second light-emitting diode (23) and
- **that** the evaluation device (28) ascertains the nitrogen dioxide concentration of the exhaust gas (1") from the signal component of the detector signal (34) resulting from the light from the first light-emitting diode (22) and the sulfur dioxide concentration from the signal component resulting from the light from the second light-emitting diode (22).

5. Gas analyser according to one of claims 2 to 4, **characterised in that** the photometers (12, 14) have two evaluation devices that communicate with one another or a common evaluation device (36) that is embodied to correct the cross-sensitivity of the ascertained sulfur dioxide concentration of the exhaust gas (1") to nitrogen dioxide with the ascertained nitrogen dioxide concentration of the exhaust gas (1").

6. Gas analyser according to claim 5, **characterised in that** the evaluation device (36) is embodied to correct the cross-sensitivity between the ascertained ozone concentration of the exhaust gas (1') and sulfur dioxide with the ascertained sulfur dioxide concentration of the exhaust gas (1").

7. Gas analyser according to one of the preceding claims, **characterised in that** a controllable bypass (19) for bypassing the oxidation device (7) is present, that, when the bypass (19) is activated, the photometer (12) measures the nitrogen dioxide concentration in the untreated exhaust gas (1) and outputs the difference from the nitrogen dioxide concentration ascertained for the treated exhaust gas (1") as the nitrogen monoxide concentration of the untreated exhaust gas (1).

8. Gas analyser according to claim 7, **characterised in that** the evaluation device (36) is embodied to correct the result (15) of the ozone concentration of the oxygen concentration of the untreated exhaust gas (1) supplied by the further photometer (14) with the ascertained nitrogen monoxide concentration of the untreated exhaust gas (1).

## Revendications

1. Analyseur de gaz pour la mesure d'oxydes d'azote et d'au moins un autre constituant d'un gaz (1) d'échappement,
- comprenant un dispositif (7) d'oxydation, qui a un générateur (8) d'ozone, une chambre (9) de réaction se trouvant dans un trajet de gaz d'échappement et une chambre (10) de chauffage se trouvant derrière elle dans le trajet du gaz d'échappement et qui est constitué pour traiter, dans la chambre (9) de réaction, le gaz (1) d'échappement par de l'ozone produit afin de transformer du monoxyde d'azote en dioxyde d'azote et pour décomposer thermiquement, dans la chambre (10) de chauffage, des oxydes d'azote et de l'ozone en excès en dioxyde d'azote et en oxygène, et
- comprenant un photomètre (12), qui se trouve dans le trajet du gaz d'échappement derrière le dispositif d'oxydation et qui est constitué pour, à l'aide de l'absorption de lumière dans le domaine de l'ultraviolet proche compris entre 350 nm et 500 nm déterminer dans le gaz (1") d'échappement traité la concentration en dioxyde d'azote et la donner comme concentration en oxyde d'azote du gaz (1) d'échappement non traité,
**caractérisé**
**en ce que** le générateur (8) d'azote comprend une source (8) de lumière ultraviolette, qui est disposée dans la chambre (9) de réaction et qui est constituée pour produire l'ozone à partir de la teneur résiduelle en oxygène du gaz (1) d'échappement, et
- **en ce qu'**un autre photomètre (14) est monté dans le trajet du gaz d'échappement entre la chambre (9) de réaction et la chambre (10) de chauffage et est constitué pour, à l'aide de l'absorption de lumière dans le domaine de l'ultraviolet moyen compris entre 220 nm et 300 nm, déterminer dans le gaz (1') d'échappement traité partiellement, sa concentration en ozone et, à l'aide d'un facteur de proportionnalité, calculer et donner la concentration en oxygène du gaz (1) d'échappement non traité.

2. Analyseur de gaz suivant la revendication 1, **caractérisé en ce que** le photomètre (12) et l'autre photomètre (14) ont chacun une chambre (21 ; 41) de mesure parcourue par le gaz (1" ; 1') d'échappement, une diode (22 ; 41) électroluminescente produisant la lumière, un détecteur (30 ; 42), un détecteur (31 ; 46) de référence, un dispositif (36) d'exploitation et un diviseur (27 ; 44) de faisceau, qui est constitué pour conduire une partie de la lumière des diodes (22 ; 40) électroluminescentes en passant, par les chambres (21 ; 41) de mesure sur le détecteur (30 ; 46) et l'autre partie de lumière sur le détecteur (31 ; 46) de référence, et **en ce que** le dispositif (36) d'exploitation référencie le signal (34 ; 43) de détecteur par le signal (35 ; 47) de référence.

3. Analyseur de gaz suivant la revendication 1 ou 2, **caractérisé en ce que** le photomètre (12) est constitué en outre pour, à l'aide de l'absorption de lumière dans le domaine de l'ultraviolet moyen compris entre 250 nm et 300 nm, déterminer dans le gaz (1") d'échappement la concentration en dioxyde de soufre du gaz (1) d'échappement et la donner.

4. Analyseur de gaz suivant la revendication 2 et 3, **caractérisé**
- **en ce que** le photomètre (12) a une deuxième diode (23) électroluminescente produisant la lumière dans le domaine de l'ultraviolet moyen compris entre 350 nm et 500 nm,
- **en ce que** le diviseur (27) de faisceau est constitué pour conduire une partie de la lumière de la deuxième diode (23) électroluminescente en passant par la chambre (21) de mesure sur le détecteur (30) et l'autre partie de la lumière sur le détecteur (31) de référence,
- **en ce qu'**il y a un dispositif (26) de commande, qui commande la première diode (22) électroluminescente et la deuxième diode (23) électroluminescente de manière différente, de manière à ce que le signal de détecteur et le signal (35) de référence contiennent chacun des proportions de signal différentes provenant de la lumière de la première diode (22) électroluminescente et de la lumière de la deuxième diode (23) électroluminescente, et
- **en ce que** le dispositif (28) d'exploitation détermine, à partir de la proportion de signal provenant de la première diode (22) électroluminescente, et du signal (34) de détecteur, la concentration en dioxyde d'azote, et, à partir de la proportion de signal provenant de la deuxième diode (22) électroluminescente, la concentration en dioxyde de soufre du gaz (1'') d'échappement.

5. Analyseur de gaz suivant les revendications 2 à 4, **caractérisé en ce que** les photomètres (12, 14) ont deux dispositifs d'exploitation communiquant entre eux, ou un dispositif (36) d'exploitation commun, qui est constitué pour corriger la sensibilité transversale de la concentration en dioxyde de soufre du gaz (1"), qui a été déterminée, par rapport au dioxyde d'azote, par la concentration en dioxyde d'azote du gaz (1") d'échappement, qui a été déterminée.

6. Analyseur de gaz suivant la revendication 5, **caractérisé en ce que** le dispositif (36) d'exploitation est constitué pour corriger la sensibilité transversale de la concentration en ozone du gaz (1') d'échappement, qui a été déterminée, par rapport au dioxyde de
soufre par la concentration en dioxyde de soufre du gaz (1") d'échappement, qui a été déterminée.

7. Analyseur de gaz suivant l'une des revendications précédentes, **caractérisé en ce qu'**il y a une dérivation (19) pouvant être commandée de contournement du dispositif (7) d'oxydation, **en ce que** le photomètre (12) mesure, lorsque la dérivation (19) est activée, la concentration en dioxyde d'azote dans le gaz (1) d'échappement non traité et donne la différence par rapport à la concentration en dioxyde d'azote, déterminée pour le gaz (1") d'échappement non traité, comme la concentration en monoxyde d'azote du gaz (1) d'échappement non traité.

8. Analyseur de gaz suivant la revendication 7, **caractérisé en ce que** le dispositif (36) d'exploitation est constitué pour corriger le résultat (15) fourni par l'autre photomètre (14) de la concentration en ozone ou de la concentration en oxygène du gaz (1) d'échappement non traité par la concentration en monoxyde d'azote du gaz (1) d'échappement non traité, qui a été déterminée.
